# EUROPÄISCHE PATENTSCHRIFT

(11) **EP 2 403 630 B1**
(45) Veröffentlichungstag und Bekanntmachung des Hinweises auf die Patenterteilung: **13.05.2015**
(21) Anmeldenummer: 10711168.4
(22) Anmeldetag: 24.02.2010
(51) Int. Cl.: B01D 69/12, B01D 67/00

(54) **MIKROSTRUKTURIERTER FORMKÖRPER MIT PERFORIERTEN TEILEN UND VERFAHREN ZU DESSEN HERSTELLUNG**
MICROSTRUCTURED MOLDED BODY COMPRISING PERFORATED PORTIONS AND METHOD FOR PRODUCING THE SAME
CORPS MOULÉ MICROSTRUCTURÉ COMPORTANT DES PARTIES PERFORÉES ET PROCÉDÉ DE FABRICATION

(30) Priorität: 27.09.2009 DE 102009044115
(43) Veröffentlichungstag der Anmeldung: 11.01.2012
(73) Patentinhaber: Technische Universität Ilmenau, 98693 Ilmenau (DE)
(72) Erfinder: SCHOBER, Andreas, 90762 Fürth (DE); HAMPL, Jörg, 99087 Erfurt (DE); WEISE, Frank, 98693 Ilmenau (DE)
(74) Vertreter: Engel, Christoph Klaus
(86) Internationale Anmeldenummer: PCT/EP2010/052353
(87) Internationale Veröffentlichungsnummer: WO 2011/035938

(56) Entgegenhaltungen:
- EP-A1- 1 721 657
- EP-A1- 2 052 844
- WO-A1-03/068612
- US-B1- 6 599 612

## Beschreibung

Die vorliegende Erfindung betrifft einen mikrostrukturierten Formkörper mit perforierten Teilen, insbesondere mit perforierten Kavitäten. Ein derartiger Formkörper kann beispielsweise zur Kultivierung von biologischen Zellen oder als Siebstruktur verwendet werden. Die Erfindung betrifft ebenso ein Verfahren zur Herstellung eines solchen Formkörpers.

Die DE 199 24 005 A1 zeigt ein Verfahren zur Herstellung von Mikrostrukturkörpern, bei welchem eine Verbundschicht aus zwei unterschiedlichen Schichten jeweils eines thermoplastischen Kunststoffs hergestellt wird. Die erste Schicht stellt eine Prägeschicht dar, während die zweite Schicht aus einem von der Trägeschicht wieder lösbarem Material besteht. Die Prägeschicht wird mit einem stempelartigen Abformwerkzeug, welches mikrostrukturierte Vorsprünge aufweist, in der Weise geprägt, dass die Vorsprünge die Prägeschicht durchstoßen und in die zweite Schicht hineinragen.

Die DE 101 34 040 A1 zeigt ein Verfahren zur Herstellung von mikrofluidischen Hohlstrukturen aus Kunststoff. In einem einzigen Prozesszyklus wird eine thermoplastische Kunststofffolie durch ein gasförmiges oder flüssiges Druckmedium zu fluidischen Mikrostrukturen thermogeformt. Gleichzeitig wird die Kunststofffolie mit einem starren oder starr flexiblen, nicht zu thermoformenden Substrat durch Einwirken von Temperatur und Druck verbunden.

Aus der DE 10 2007 050 976 A1 und der EP 2 052 844 A1 ist ein Verfahren zur Umformung einer Folie bekannt, bei welchem die umzuformende Folie fest mit einer Formkulisse, die mindestens einen Durchbruch aufweist, verbunden wird. Anschließend werden Bereiche der umzuformenden Folie einer physikalischen oder chemischen Modifikation unterworfen. Die derart modifizierte und mit der Formkulisse verbundene Folie wird in ein Formwerkzeug eingelegt, und dort mit einem Druckmedium beaufschlagt, welches die Folie zumindest teilweise in den Durchbruch der Formkulisse einformt.

Die US 6,599,612 B1 zeigt ein Verfahren zur Ausbildung einer durch Öffnungen gebildeten Matrix in einem weichen und elastischen Gewebe. Die Öffnungen werden dadurch geschaffen, dass eine Folie erwärmt wird und an den vorgesehenen Stellen örtlich begrenzt mit einem Druck beaufschlagt wird. Hierfür wird die Folie auf formenden Strukturen angeordnet. Die formenden Strukturen sind als rotierende Trommeln ausgebildet, über welche die Folie gefördert wird.

Die DE 10 2004 035 267 B3 zeigt einen mikrostrukturierten Formkörper und ein Verfahren zu dessen Herstellung. Der Formkörper besteht aus einer Folie, in die mindestens eine Hohlstruktur eingebracht ist. Die Hohlstruktur weist bevorzugt eine Ω-Struktur auf. Der gesamte Formkörper, d. h. sowohl die Folie als auch die Hohlstrukturen weisen eine Vielzahl von Poren auf, deren Durchmesser bevorzugt einen Wert zwischen 10 nm und 10 µm annimmt. Die Poren sind statistisch über den ganzen Formkörper, d. h. über die Folie und die Hohlstrukturen verteilt, wobei es vorkommen kann, dass sich einzelne Poren überlappen. Die Fig. 7 der DE 10 2004 035 267 B3 lässt deutlich erkennen, dass die Poren sowohl in der Porenstruktur als auch in unverformten Bereichen der Folie ausgebildet sind. Zur Einbringung der Poren wird die Folie mit einer ionisierenden Strahlung beaufschlagt. Die Ionen hinterlassen latent vorhandene Spuren, welche durch einen Ätzvorgang zu Poren führen.

Ein Nachteil dieser Lösung besteht zunächst darin, dass die Poren im gesamten Formkörper ausgebildet sind. Dies kann beispielsweise zu parasitären Strömungen bei der Kultivierung von biologischen Zellen führen. Um zu verhindern, dass die Poren im gesamten Formkörper gebildet werden, wird ferner vorgeschlagen, eine Maske zu verwenden, um die Bestrahlung mit Ionen auf bestimmte Bereiche der Folie zu beschränken. Die Maske muss die mit Poren zu versehenden Bereiche exakt abbilden, wofür ein erhöhter Aufwand erforderlich ist.

Die Aufgabe der Erfindung besteht ausgehend vom Stand der Technik darin, einen mikrostrukturierten Formkörper sowie ein Verfahren zu dessen Herstellung bereitzustellen, welcher nur teilweise perforiert und aufwandsarm herstellbar ist.

Die genannte Aufgabe wird durch ein Verfahren zur Herstellung eines mikrostrukturierten Formkörpers mit perforierten Teilen gemäß dem beigefügten Anspruch 1 gelöst. Die Aufgabe wird weiterhin durch einen mikrostrukturierten Formkörper gemäß dem beigefügten nebengeordneten Anspruch 4 gelöst.

Das erfindungsgemäße Verfahren dient der Herstellung eines mikrostrukturierten Formkörpers, welcher beispielsweise zur Kultivierung von biologischen Zellen oder als Siebstruktur verwendet werden kann. Der herzustellende Formkörper weist perforierte Teile auf, d. h. nur einige Teile bzw. nur einige Bereiche des Formkörpers weisen Poren auf. Das erfindungsgemäße Verfahren umfasst zunächst einen Schritt, bei welchem eine verformbare erste Folie mit einer Dicke von weniger als 1 mm, bevorzugt weniger als 100 µm bereitgestellt wird. Weiterhin wird eine zweite Folie bereitgestellt, deren Dicke bevorzugt mehrfach so groß wie die Dicke der ersten Folie ist. Die zweite Folie weist durchgängige Ausnehmungen auf, deren Durchmesser kleiner als 2 mm, bevorzugt kleiner als 1 mm sind. Die Ausnehmungen sind im einfachsten Fall durch Löcher mit einem kreisförmigen Querschnitt gebildet, die matrixartig angeordnet sind. Die Ausnehmungen bestimmen die Form und Größe von Kavitäten oder ähnlichen Strukturen, welche der herzustellende Formkörper aufweisen wird. Die Ausnehmungen können beispielsweise auch einen rechteckförmigen Querschnitt besitzen, um eine Mikrostruktur in Form eines Kanals zu bilden. In einem weiteren Schritt des erfindungsgemäßen Verfahrens werden Poren in der ersten Folie erzeugt. Bei den Poren handelt es sich um durchgängige Öffnungen, durch die beispielsweise ein Gas oder eine Flüssigkeit durch die erste Folie treten kann. Die Poren weisen einen vielfach kleineren Durchmesser als die Ausnehmungen auf. Der Durchmesser der Poren beträgt zwischen 10 nm und 10 µm. In einem weiteren Schritt des erfindungsgemäßen Verfahrens wird die erste Folie über der zweiten Folie angeordnet. Hierdurch bedeckt die erste Folie auch die Ausnehmungen, welche in der zweiten Folie vorhanden sind. Die beiden Folien sind bevorzugt passgenau und überdeckend zueinander, beispielsweise in einem Formwerkzeug anzuordnen. Diejenigen Bereiche der ersten Folie, welche sich über oder auch um eine der Ausnehmungen der zweiten Folie befinden, werden in einem weiteren Schritt in die jeweilige Ausnehmung eingeformt, sodass die verformten Bereiche Kavitäten ausbilden. Weiterhin ist die erste Folie mit der zweiten Folie zu verbinden, sodass ein einziger Formkörper ausgebildet wird, in welchem die Kavitäten Mikrostrukturen bilden, und bei welchem die durchgängigen Poren nur in den verformten Bereichen der ersten Folie vorhanden sind, während diejenigen Bereiche der zweiten Folie, welche die Kavitäten umgeben, undurchlässig sind. Das Verbinden der ersten Folie mit der zweiten Folie kann je nach Ausführungsform des erfindungsgemäßen Verfahrens in unterschiedlichen Verfahrensstadien erfolgen, beispielsweise vor oder nach dem Verformen der zu verformenden Bereiche der ersten Folie. Das Verbinden der ersten Folie mit der zweiten Folie erfolgt durch ein Diffusionsschweißen, ein Laser-Schweißen, ein Ultraschallschweißen, ein Laminieren mit einer dazwischen befindlichen Klebeschicht oder durch ein anderes Verfahren zum Verbinden der Folien.

Ein besonderer Vorteil des erfindungsgemäßen Verfahrens besteht darin, dass durch die Verwendung von zwei Folien ein mikrostrukturierter Formkörper erzeugt wird, welcher nur im Bereich der Kavitäten durchgängige Poren aufweist. Hingegen weist der Formkörper außerhalb der Kavitäten keine durchgängigen Poren auf, sodass er dort nahezu oder vollständig undurchlässig ist.

Die Poren werden vor dem Verformen der ersten Folie erzeugt. Die Poren werden sowohl in den zu verformenden Bereichen als auch in nicht zu verformenden Bereichen der ersten Folie erzeugt. Diejenigen Poren, welche sich in nicht zu verformenden Bereichen der ersten Folie befinden, werden in einem spätern Schritt des erfindungsgemäßen Verfahrens, nämlich durch das Verbinden der ersten Folie mit der zweiten Folie durch die zweite Folie wieder verschlossen. Der entstehende mikrostrukturierte Formkörper weist somit nur im Bereich der Kavitäten, welche durch die verformten Bereiche der ersten Folie gebildet sind, Poren auf. In den übrigen Bereichen ist der Formkörper nicht durchlässig.

Bei einer ersten Ausführungsform außerhalb der Erfindung umfasst das Erzeugen der Poren zunächst einen Teilschritt, bei welchem die erste Folie mit einer ionisierenden Strahlung bestrahlt wird. Dieses Bestrahlen erfolgt vor dem Anordnen der ersten Folie über der zweiten Folie, d. h. in einem Moment, in welchem die erste Folie noch separat vorliegt. Die Bestrahlung der ersten Folie mit einer ionisierenden Strahlung führt zu Ionendurchschüssen in der ersten Folie. Die Ionendurchschüsse bewirken, dass eine Struktur des Werkstoffs der ersten Folie jeweils im Bereich des Ionendurchschusses über die gesamte Dicke der ersten Folie zerstört wird. Die Bereiche der Ionendurchschüsse werden auch als latente Störstellen bezeichnet. Das Erzeugen der Poren umfasst weiterhin einen Teilschritt, welcher erst nach dem Verbinden der ersten Folie mit der zweiten Folie erfolgt. Dieser Schritt wird somit auf den mikrostrukturierten Formkörper angewendet und umfasst das Ätzen des mikrostrukturierten Formkörpers, wodurch sich aus den Ionendurchschüssen durchgängige Poren in den verformten Bereichen der ersten Folie entstehen. Die Ionendurchschüsse in den unverformten Bereichen der ersten Folie können ggf. auch zu Poren in der ersten Folie führen, jedoch bleiben diese Poren durch die darunter angeordnete zweite Folie jedenfalls verschlossen, sodass dort keine bezogen auf den mikrostrukturierten Formkörper durchgängige Poren ausgebildet werden.

Bei einer zweiten Ausführungsform außerhalb der Erfindung werden die Poren erst nach dem Verbinden der ersten Folie und der zweiten Folie dadurch erzeugt, dass ein Perforationsverfahren zur Erzeugung von Poren auf den gesamten mikrostrukturierten Formkörper angewendet wird, wobei Parameter des Perforationsverfahrens so bemessen werden, dass Poren in der zweiten Folie nicht entstehen. Bezogen auf den gesamten mikrostrukturierten Formkörper entstehen die Poren somit nur in den verformten Bereichen der ersten Folie. Außerhalb der Ausnehmungen der zweiten Folie, d. h. dort, wo Bereiche der ersten Folie und der zweiten Folie übereinander angeordnet sind, entstehen zumindest keine durchgängigen Poren.

Das Perforationsverfahren zur Erzeugung von Poren begrenzt auf die erste Folie umfasst bevorzugt zunächst einen Teilschritt, bei welchem der mikrostrukturierte Formkörper mit einer ionisierenden Strahlung bestrahlt wird. Die Intensität der ionisierenden Strahlung wird dabei so bemessen, dass nur in der ersten Folie Ionendurchschüsse entstehend. Anschließend wird der mikrostrukturierte Formkörper geätzt, wodurch aus den Ionendurchschüssen durchgängige Poren entstehen. Da in der zweiten Folie keine Ionendurchschüsse, sondern allenfalls Ioneneinschüsse vorhanden sind, werden dort keine durchgängigen Poren entstehen.

Die Schritte des Anordnens der ersten Folie über der zweiten Folie, des Verformens der ersten Folie und des Verbindens der ersten Folie mit der zweiten Folie erfolgen bevorzugt in einem Formwerkzeug und umfassen zunächst einen Teilschritt, bei welchem ein Thermoplast als Material für die erste Folie verwendet wird. Für die zweite Folie wird ein Material verwendet, welches bei einer Glasübergangstemperatur des Materials der ersten Folie noch weitgehend unverformbar ist. Es kann sich beispielsweise ebenfalls um einen Thermoplast handeln, wobei die Glasübergangstemperatur des Thermoplasts der zweiten Folie höher als die Glasübergangstemperatur des Thermoplasts der ersten Folie zu wählen ist. Die erste Folie ist über der zweiten Folie zwischen einer ersten Formhälfte und einer zweiten Formhälfte anzuordnen. Die erste Folie ist dabei zwischen der zweiten Formhälfte und der zweiten Folie anzuordnen. Zumindest die erste Folie ist mindestens bis zur Glasübergangstemperatur des Thermoplasts der ersten Folie zu erwärmen, wodurch die erste Folie thermoplastisch verformbar wird. Um diese Verformung zu bewirken, wird zwischen der ersten Formhälfte und der zweiten Folie ein Unterdruck erzeugt, der durch die Ausnehmungen in der zweiten Folie auch auf die zu verformenden Bereiche der ersten Folie wirkt. Die zu verformenden Bereiche sind wie die gesamte erste Folie thermoplastisch verformbar, sodass sie in die Ausnehmungen in der zweiten Folie geformt werden. Der Unterdruck ist dadurch gekennzeichnet, dass er geringer als ein Druck zwischen der ersten Folie und der zweiten Formhälfte ist. Folglich kann der Unterdruck zwischen der ersten Formhälfte und der zweiten Folie beispielsweise dadurch erzeugt werden, dass die Luft aus dem Bereich zwischen der ersten Formhälfte und der zweiten Folie herausgepumpt wird. Der Unterdruck kann aber auch dadurch erzeugt werden, dass der Druck zwischen der ersten Folie und der zweiten Formhälfte dadurch erhöht wird, dass Druckluft in den Bereich zwischen der ersten Folie und der zweiten Formhälfte eingeleitet wird. Die zu verformenden Bereiche der ersten Folie werden in die Ausnehmungen hineingezogen und legen sich an die Innenseite der Ausnehmungen an. Die zu verformenden Bereiche werden bevorzugt soweit durch die Ausnehmungen gezogen, dass sie durch die Ausnehmungen hindurch und aus den Ausnehmungen herausragen. In einem weiteren Teilschritt dieser Ausführungsform des erfindungsgemäßen Verfahrens werden die erste Formhälfte und die zweite Formhälfte mit den beiden dazwischen befindlichen Folien zusammengepresst, wodurch die beiden Folien infolge eines Diffusionsschweißvorganges miteinander verbunden werden. Dieser Teilschritt erfolgt bevorzugt isostatisch. Der Vorgang kann auch vor dem Verformen der zu verformenden Bereiche der ersten Folie durchgeführt werden. In diesem Fall sind die erste Folie und die zweite Folie als Einheit zwischen die erste Formhälfte und die zweite Formhälfte anzuordnen. In einem weiteren Teilschritt kühlen die beiden Folien ab, wodurch ein einziger mikrostrukturierter Formkörper geschaffen wird. Der mikrostrukturierte Formkörper ist aus den beiden Formhälften herauszunehmen.

Der Schritt des Erwärmens der ersten Folie erfolgt bevorzugt dadurch, dass die erste und die zweite Formhälfte erwärmt werden. Hierdurch erwärmen sich auch die zwischen der ersten Formhälfte und der zweiten Formhälfte befindlichen Folien. Dabei ist die zweite Formhälfte bevorzugt stärker zu erwärmen, um zu erreichen, dass die erste Folie eine höhere Temperatur als die zweite Folie erfährt. Die zweite Folie ist nicht mehr als unvermeidbar zu erwärmen, um zu gewährleisten, dass diese weitgehend unverformbar bleibt.

Bei einer besonderen Ausführungsform des erfindungsgemäßen Verfahrens wird beim Anordnen der ersten Folie über der zweiten Folie zwischen der ersten Formhälfte und der zweiten Formhälfte weiterhin eine zu verformende Opferfolie zwischen der ersten Folie und der zweiten Formhälfte angeordnet. Die Verwendung einer Opferfolie ist insbesondere dann bevorzugt, wenn die Poren in die erste Folie bereits vor der Verformung der zu verformenden Bereiche der ersten Folie erzeugt wurden. Durch die Opferfolie wird das Erzeugen eines Unterdrucks zwischen der ersten Formhälfte und der zweiten Folie erleichtert, da keine Luft durch die von der Opferfolie bedeckten Poren in diesen Bereich strömen kann. Die verformte Opferfolie ist nach dem Herausnehmen des mikrostrukturierten Formkörpers aus den beiden Formhälften von dem mikrostrukturierten Formkörper zu trennen.

Der erfindungsgemäße mikrostrukturierte Formkörper umfasst zunächst einen folienartigen Grundkörper, aus welchem er im einfachsten Fall vollständig gebildet ist. Der erfindungsgemäße mikrostrukturierte Formkörper kann aber auch zusätzliche Elemente, beispielsweise zum Einfassen des folienartigen Grundkörpers besitzen. Der folienartige Grundkörper umfasst eine erste Folienschicht und eine darunter befindliche zweite Folienschicht. Die beiden Folienschichten sind fest miteinander verbunden und bilden eine einzige Einheit aus. Die zweite Folienschicht weist Ausnehmungen mit einem Durchmesser von weniger als 2 mm, bevorzugt weniger als 1 mm auf. Die Ausnehmungen sind durch verformte Bereiche der ersten Folienschicht blasenartig ausgeformt, wodurch innerhalb der verformten Bereiche Kavitäten oder ähnliche Strukturen ausgebildet sind. Erfindungsgemäß weisen zumindest einige der verformten Bereiche der ersten Folienschicht Poren auf, während zumindest Bereiche des folienartigen Grundkörpers außerhalb der Ausnehmungen undurchlässig sind. Bevorzugt ist die gesamte zweite Folienschicht abgesehen von den Ausnehmungen undurchlässig. Bevorzugt ist die gesamte erste Folienschicht außerhalb der verformten Bereiche undurchlässig. Jedenfalls ist bevorzugt der gesamte mikrostrukturierte Formkörper abgesehen von den verformten Bereichen der ersten Folienschicht in den Ausnehmungen der zweiten Folienschicht undurchlässig. Dabei ist es ohne Belang, ob die erste Folienschicht in unverformten Bereichen Poren aufweist, da diese Poren jedenfalls durch die zweite Folienschicht verschlossen sind.

Ein besonderer Vorteil des erfindungsgemäßen mikrostrukturierten Formkörpers besteht darin, dass er in einem hohen Maße an die vorgesehene Anwendung angepasst sein kann, insbesondere dadurch, dass er nur dort Poren aufweist, wo diese erforderlich sind, und folglich keine Poren dort aufweist, wo sie u. U. die vorgesehene Anwendung beeinträchtigen.

Die zweite Folienschicht ist bevorzugt mehr als doppelt so dick wie die erste Folienschicht. Die zweite Folienschicht bestimmt die mechanische Stabilität des mikrostrukturierten Formkörpers, während die erste Folienschicht eine Hülle für die Kavitäten bildet, deren durch die Poren bedingte Durchlässigkeit eine wesentliche Eigenschaft des erfindungsgemäßen mikrostrukturierten Formkörpers darstellt. Folglich ist es von Vorteil, wenn die zweite Folienschicht weitaus dicker als die erste Folienschicht ist. Beispielsweise kann als Dicke für die erste Folie ein Maß von 50 µm gewählt werden, während als Dicke für die zweite Folie ein Maß von 300 µm gewählt wird.

Die Tiefe der Kavitäten ist bevorzugt jeweils mehr als doppelt so groß wie der Durchmesser der durch die jeweilige Kavität ausgeformten Ausnehmung. Ein besonderer Vorteil des erfindungsgemäßen mikrostrukturierten Formkörpers besteht nämlich darin, dass die Tiefe der Kavitäten in einem weiten Bereich frei gewählt werden kann. Insbesondere kann die Tiefe der Kavitäten groß im Vergleich zum Durchmesser der Kavität bzw. dem Durchmesser der durch die jeweilige Kavität ausgeformten Ausnehmung gewählt werden. Die Tiefe der Kavitäten kann beispielsweise so gewählt werden, dass sie der Dicke der zweiten Folienschicht gleicht. In diesem Fall ragen die Kavitäten gerade nicht aus der zweiten Folienschicht heraus. Die Tiefe der Kavitäten kann aber auch so gewählt werden, dass sie deutlich größer als die Dicke der zweiten Folienschicht ist. In diesem Fall ragen die Kavitäten aus der zweiten Folienschicht heraus. Dies führt zu dem Vorteil, dass ein großer Teil der verformten Bereiche der ersten Folienschicht mit den darin vorhandenen Poren aus der zweiten Folienschicht herausragen und so eine große Menge Flüssigkeit bzw. Gas durch die Poren hindurchströmen kann. Diese Ausführungsform weist weiterhin den Vorteil auf, dass die Kavitäten aufgrund Ihrer Tiefe eine hohe Aufnahmekapazität besitzen. Zudem ist der in den Kavitäten anzuordnender Inhalt, wie beispielsweise biologische Zellen, in einem höheren Maße gegen ein Herausfallen aus den Kavitäten gesichert, als dies bei Kavitäten mit einer geringen Tiefe der Fall ist.

Weitere Vorteile, Weiterbildungen und Einzelheiten der Erfindung ergeben sich aus der nachfolgenden Beschreibung bevorzugter Ausführungsformen, unter Bezugnahme auf die Zeichnung. Es zeigen:
- Fig. 1:: eine Ausführungsform eines mikrostrukturierten Formkörpers in einer Schnittansicht;
- Fig. 2:: einen verformten Bereich des in Fig. 1 gezeigten Formkörpers;
- Fig. 3:: einen unverformten Bereich des in Fig. 1 gezeigten Formkörpers;
- Fig. 4:: einen ersten Schritt einer Ausführungsform eines Verfahrens zur Herstellung eines mikrostrukturierten Formkörpers;
- Fig. 5:: einen zweiten Schritt der Ausführungsform des Verfahrens;
- Fig. 6:: einen dritten Schritt der Ausführungsform des Verfahrens;
- Fig. 7:: einen vierten Schritt der Ausführungsform des Verfahrens;
- Fig. 8:: einen fünften Schritt der Ausführungsform des Verfahrens;
- Fig. 9:: einen sechsten Schritt der Ausführungsform des Verfahrens;
- Fig. 10:: einen siebten Schritt der Ausführungsform des Verfahrens;
- Fig. 11:: einen ersten Schritt einer ersten bevorzugten Ausführungsform des erfindungsgemäßen Verfahrens zur Herstellung eines mikrostrukturierten Formkörpers;
- Fig. 12:: einen zweiten Schritt der ersten bevorzugten Ausführungsform des erfindungsgemäßen Verfahrens;
- Fig. 13:: ein Detail eines in Fig. 12 gezeigten Halbzeuges;
- Fig. 14:: einen dritten Schritt der ersten bevorzugten Ausführungsform des erfindungsgemäßen Verfahrens;
- Fig. 15:: einen vierten Schritt der ersten bevorzugten Ausführungsform des erfindungsgemäßen Verfahrens;
- Fig. 16:: einen fünften Schritt der ersten bevorzugten Ausführungsform des erfindungsgemäßen Verfahrens;
- Fig. 17:: einen sechsten Schritt der ersten bevorzugten Ausführungsform des erfindungsgemäßen Verfahrens;
- Fig. 18:: einen ersten Schritt einer zweiten bevorzugten Ausführungsform des erfindungsgemäßen Verfahrens zur Herstellung eines mikrostrukturierten Formkörpers;
- Fig. 19:: einen zweiten Schritt einer zweiten bevorzugten Ausführungsform des erfindungsgemäßen Verfahrens;
- Fig. 20:: ein Detail eines in Fig. 19 gezeigten Halbzeugs;
- Fig. 21:: einen dritten Schritt der zweiten bevorzugten Ausführungsform des erfindungsgemäßen Verfahrens;
- Fig. 22:: einen vierten Schritt der zweiten bevorzugten Ausführungsform des erfindungsgemäßen Verfahrens,
- Fig. 23:: einen fünften Schritt der zweiten bevorzugten Ausführungsform des erfindungsgemäßen Verfahrens,
- Fig. 24:: einen sechsten Schritt der zweiten bevorzugten Ausführungsform des erfindungsgemäßen Verfahrens; und
- Fig. 25:: einen siebten Schritt der zweiten bevorzugten Ausführungsform des erfindungsgemäßen Verfahrens.

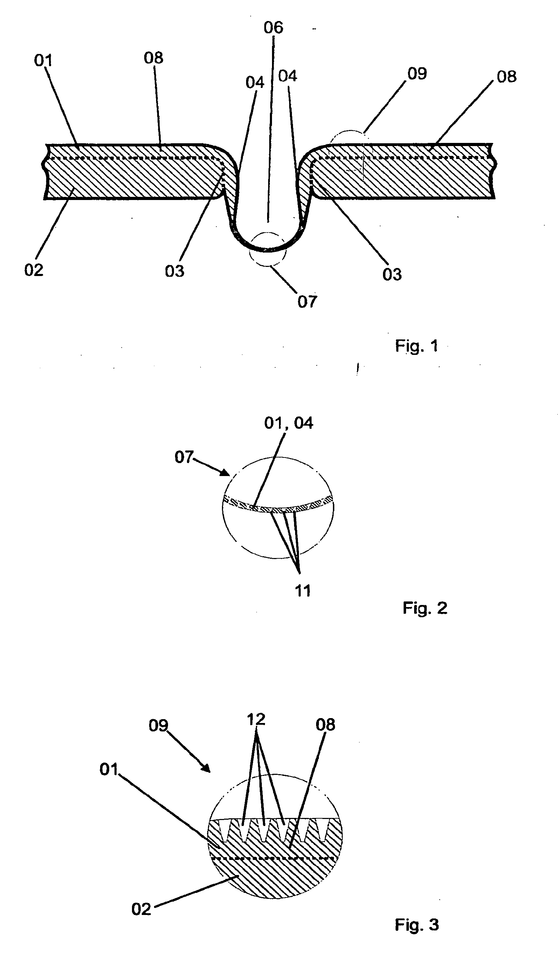

Fig. 1 zeigt eine Ausführungsform eines mikrostrukturierten Formkörpers in einer Schnittansicht, wobei lediglich ein repräsentativer Ausschnitt des mikrostrukturierten Formkörpers gezeigt ist. Der mikrostrukturierte Formkörper umfasst eine erste Folienschicht 01 aus einem Polycarbonat, welche eine Dicke von etwa 50 µm aufweist. Der Formkörper umfasst weiterhin eine zweite Folienschicht 02 aus einem Polycarbonat, welche eine Dicke von etwa 300 µm besitzt. Die erste Folienschicht 01 und die zweite Folienschicht 02 sind fest miteinander verbunden und bilden eine Einheit. Die zweite Folienschicht 02 weist eine durchgängige Ausnehmung 03 mit einem kreisförmigen Querschnitt auf. Der Durchmesser der Ausnehmung 03 beträgt etwa 500 µm. Die zweite Folienschicht 02 weist eine Vielzahl weiterer Ausnehmungen (nicht gezeigt) auf, die in gleicher Weise wie die Ausnehmung 03 ausgeführt sind. Sämtliche Ausnehmungen in der zweiten Folienschicht 02 sind matrixförmig angeordnet. Die erste Folienschicht 01 ist im Bereich der Ausnehmung 03 verformt. Es ist ein verformter Bereich 04 der ersten Folienschicht 01 ausgebildet, welcher die Ausnehmung 03 blasenartig ausfüllt. Dieses Ausfüllen ist zunächst dadurch charakterisiert, dass die erste Folienschicht 01 im verformten Bereich 04 an die Wandung der Ausnehmung 03 anliegend angeordnet ist. Da die Dicke der ersten Folienschicht 01 deutlich kleiner als der Durchmesser der Ausnehmung 03 ist, ist im verformten Bereich 04 der ersten Folienschicht 01 innerhalb der Ausnehmung 03 ein Hohlraum in Form einer Kavität 06 ausgebildet. Der verformte Bereich 04 erstreckt sich über die gesamte Dicke der zweiten Folienschicht 02 und über diese Dicke hinaus, sodass der verformte Bereich 04 aus der Ausnehmung 03 der zweiten Folienschicht 02 herausragt. Insbesondere in dem aus der Ausnehmung 03 herausragenden Teil des verformten Bereichs 04 weist die erste Folienschicht 01 eine geringere Dicke als in denjenigen Bereichen auf, in welchen die erste Folienschicht 01 nicht verformt ist. Ein Kreis kennzeichnet einen verformten Bereich 07, in welchem die erste Folienschicht 01 eine minimale Dicke aufweist. Der verformte Bereich 07 ist in Fig. 2 im Detail gezeigt.

In Bereichen 08 außerhalb der Ausnehmung 03 ist die erste Folienschicht 01 unverformt. Ein Kreis kennzeichnet einen Teilbereich 09 eines der unverformten Bereiche 08, welcher in Fig. 3 im Detail gezeigt ist.

Fig. 2 zeigt den Bereich 07 des in Fig. 1 gezeigten mikrostrukturierten Formkörpers im Detail. Die erste Folienschicht 01 weist im verformten Bereich 04, insbesondere in einem Teilbereich, in welchem die erste Folienschicht 01 eine minimale Dicke aufweist, durchgängige Poren 11 auf, welche einen Durchmesser von etwa 2 µm besitzen. Durch die Poren 11 können Gase oder Flüssigkeiten hindurchströmen.

Fig. 3 zeigt den Bereich 09 des in Fig. 1 gezeigten mikrostrukturierten Formkörpers im Detail. Es sind wiederum die erste Folienschicht 01 und die zweite Folienschicht 02 in einer Schnittansicht dargestellt. Die erste Folienschicht 01 weist in den unverformten Bereichen 08 Krater 12 auf. Die Krater 12 sind durch Ansätze von Poren gebildet. Jedenfalls sind die Krater 12 nicht durchgängig ausgebildet. Selbst wenn die Krater 12 bis zur zweiten Folienschicht 02 reichen, bilden sie bezogen auf den Formkörper keine durchgängige Öffnung aus.

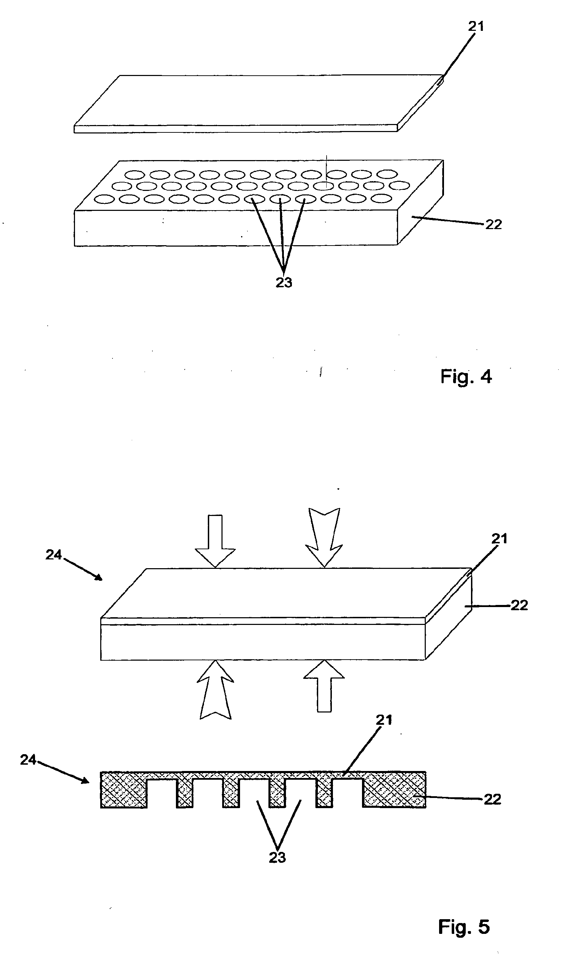

Fig. 4 zeigt einen ersten Schritt einer Ausführungsform eines Verfahrens zur Herstellung eines mikrostrukturierten Formkörpers. Bei diesem ersten Schritt werden eine verformbare erste Folie 21 und eine zweite Folie 22 bereitgestellt. Bei der ersten Folie 21 handelt es sich um eine Polycarbonatfolie mit einer Dicke von etwa 50 µm. Als Material für die erste Folie 21 kommen aber auch andere Materialien in Betracht, welche sich geeignet verformen lassen, insbesondere auch andere thermoplastisch verformbare Polymere. Die Dicke der ersten Folie 21 muss so gewählt werden, dass sie nach einer Verformung Mikrostrukturen, insbesondere Kavitäten ausbilden kann. Die zweite Folie 22 ist mit einer Dicke von etwa 300 µm wesentlich dicker als die erste Folie 21. Als Material für die zweite Folie 22 kann ebenfalls ein Polycarbonat gewählt werden. Die zweite Folie 22 darf weit weniger verformbar als die erste Folie 21 sein, da die zweite Folie 22 eine Form für die erste Folie 21 bilden wird. Als Material für die zweite Folie 22 kann aber ebenfalls ein thermoplastisch verformbares Polymer gewählt werden, insofern die Glasübergangstemperatur des Materials der zweiten Folie 22 höher als die Glasübergangstemperatur des Materials der ersten Folie 21 gewählt wird. Der Unterschied der beiden Glasübergangstemperaturen ist möglichst groß zu wählen. Geeignete Werkstoffe hierfür sind dem Fachmann bekannt. Die zweite Folie 22 weist eine Vielzahl an Ausnehmungen 23 auf, die matrixartig angeordnet sind. Die Ausnehmungen 23 weisen einen kreisförmigen Querschnitt auf und besitzen einen Durchmesser von etwa 500 µm. Die Ausnehmungen 23 können auch deutlich kleiner ausgeführt werden, wofür jedoch die Dicke der ersten Folie 21 entsprechend dünner zu wählen ist. Die Ausnehmungen 23 sind senkrecht zur Ausdehnung der zweiten Folie 22 angeordnet und durchgängig ausgebildet. Die Ausnehmungen 23 können beispielsweise durch ein Laserstrahlbohren, durch ein Feinstanzen oder durch ein Prägen in die zweite Folie 22 eingebracht werden.

Die erste Folie 21 wurde zuvor einer Bestrahlung mit Schwerionen ausgesetzt. Die Schwerionen wiesen eine derartige Energie auf, dass die erste Folie 21 durch die Schwerionen durchschossen wurde. Daher weist die gesamte erste Folie 21 statistisch verteilte Ionendurchschüsse (nicht dargestellt) auf.

Fig. 5 zeigt einen zweiten Schritt der Ausführungsform des Verfahrens, welcher in einem Halbzeug 24 resultiert. Das Halbzeug 24 ist in einer perspektivischen Ansicht und in einer Schnittansicht dargestellt. Bei diesem zweiten Schritt des Verfahrens werden die erste Folie 21 und die zweite Folie 22 durch einen Diffusionsschweißvorgang miteinander verbunden. Für die Durchführung des Diffusionsschweißens werden die erste Folie 21 und die zweite Folie 22 unter der Einwirkung von Wärme einem isostatischen Druck ausgesetzt. Der Druck wird isostatisch erzeugt, um ein Verformen der ersten Folie 21 im Bereich der Ausnehmungen 23 zu verhindern. Nachdem das Diffusionsschweißen abgeschlossen ist, bilden die erste Folie 21 und die zweite Folie 22 das Halbzeug 24 aus.

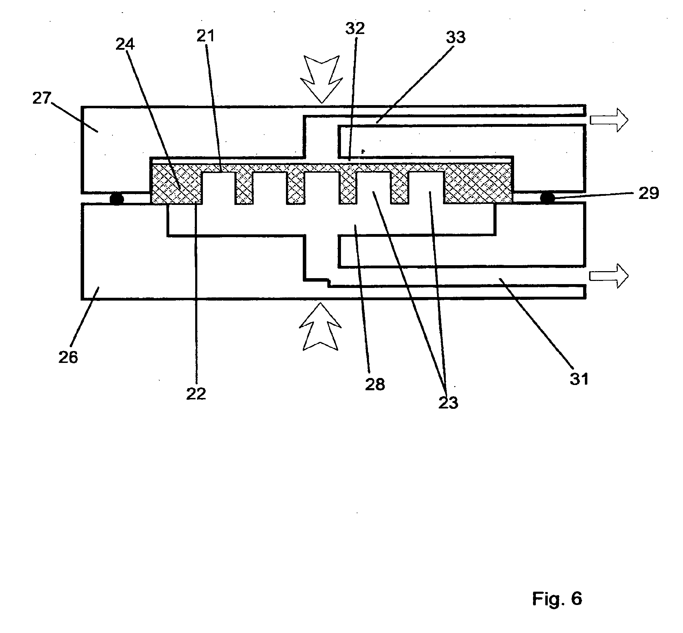

Fig. 6 zeigt einen dritten Schritt der Ausführungsform des Verfahrens. Bei diesem dritten Schritt wird das Halbzeug 24 zwischen eine erste Formhälfte 26 und eine zweite Formhälfte 27 eines Formwerkzeuges gelegt. Die erste Formhälfte 26 und die zweite Formhälfte 27 bestehen aus einem festen temperaturbeständigen Material, wie beispielsweise einem Metall oder Glas. Das Halbzeug 24 wird so zwischen der ersten Formhälfte 26 und der zweiten Formhälfte 27 angeordnet, dass die erste Folie 21 zwischen der zweiten Folie 22 und der zweiten Formhälfte 27 angeordnet ist. Folglich ist die zweite Folie 22 zwischen der ersten Folie 21 und der ersten Formhälfte 26 angeordnet. Zwischen der zweiten Folie 22 und der ersten Formhälfte 26 ist ein erster Hohlraum 28 ausgebildet, welcher am Übergang zur zweiten Formhälfte 27 durch Dichtelemente 29 abgedichtet ist. Der erste Hohlraum 28 umfasst weiterhin die Ausnehmungen 23, sodass der erste Hohlraum 28 im Bereich der Ausnehmungen 23 durch die erste Folie 21 abgeschlossen ist. Durch die erste Formhälfte 26 führt ein erster Kanal 31 von außerhalb der ersten Formhälfte 26 in den ersten Hohlraum 28. Zwischen der ersten Folie 21 und der zweiten Formhälfte 27 ist ein zweiter Hohlraum 32 ausgebildet, zu welchem ein zweiter Kanal 33 von außerhalb der zweiten Formhälfte 27 führt.

In dem gezeigten dritten Schritt der Ausführungsform des Verfahrens wird über den ersten Kanal 31 und über den zweiten Kanal 33 die Luft aus dem ersten Hohlraum 28 und aus dem zweiten Hohlraum 32 herausgesaugt, sodass im ersten Hohlraum 28 und im zweiten Hohlraum 32 jeweils ein technisches Vakuum vorhanden ist. Gleichzeitig wird Wärme auf die erste Formhälfte 26 und auf die zweite Formhälfte 27 übertragen, um das Halbzeug 24 zu erwärmen. Dabei wird die zweite Formhälfte 27 auf eine Temperatur erwärmt, welche etwa 5 Kelvin größer als die Temperatur der ersten Formhälfte 26 ist. Dadurch soll gewährleistet werden, dass die erste Folie 21 eine höhere Temperatur als die zweite Folie 22 annimmt.

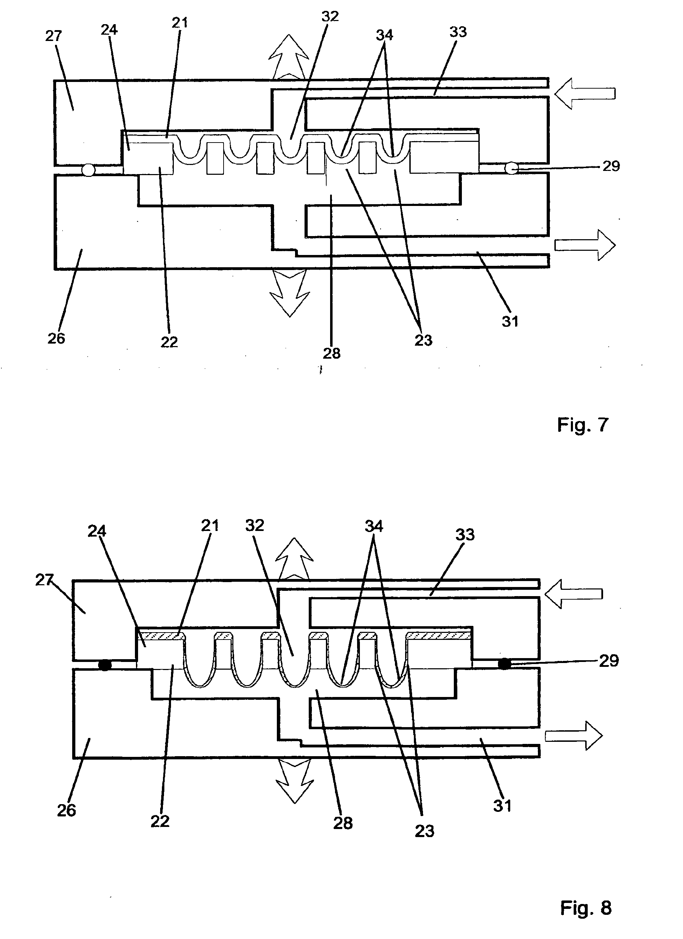

Fig. 7 zeigt einen vierten Schritt der Ausführungsform des Verfahrens. Bei diesem vierten Schritt wird das im zweiten Hohlraum 32 vorhandene technische Vakuum abgebaut und stattdessen über den zweiten Kanal 33 ein Gas mit einem hohen dynamischen Druck in Form eines Druckstoßes eingeleitet. Gleichzeitig wird das im ersten Hohlraum 28 vorhandene technische Vakuum weiter aufrechterhalten. Folglich weist der erste Hohlraum 28 gegenüber dem zweiten Hohlraum 32 einen großen Unterdruck auf. Dieser Unterdruck führt dazu, dass sich die erste Folie 21, welche bis oberhalb ihrer Glasübergangstemperatur, jedoch unterhalb der Glasübergangstemperatur der zweiten Folie 22 erwärmt wurde, in den Bereichen der Ausnehmungen 23 verformt. Es entstehen verformte Bereiche 34 der ersten Folie 21, welche die Ausnehmungen 23 zunehmend ausfüllen. Der Verformungsvorgang der ersten Folie 21 ist vergleichbar mit einem Tiefziehen. Die zweite Folie 22 verformt sich unter dem Einfluss des im zweiten Hohlraum 32 vorhandenen Druckstoßes nicht, da zum einen die Glasübergangstemperatur der zweiten Folie 22 nicht erreicht ist und zum anderen die zweite Folie 22 aufgrund ihrer größeren Dicke ohnehin deutlich stabiler ist. In diesem vierten Schritt des Verfahrens wurde die Zuführung von Wärme auf die erste Formhälfte 26 und die zweite Formhälfte 27 bereits beendet.

Fig. 8 zeigt einen fünften Schritt der Ausführungsform des Verfahrens. Bei dem gezeigten fünften Schritt wurde der im zweiten Hohlraum 32 vorhandene Druckstoß weiter aufrechterhalten. Dies führte dazu, dass die erste Folie 21 in den verformten Bereichen 34 weiter verformt wurde. Die verformten Bereiche 34 erstrecken sich nunmehr über die gesamten Ausnehmungen 23 und treten aus diesen heraus.

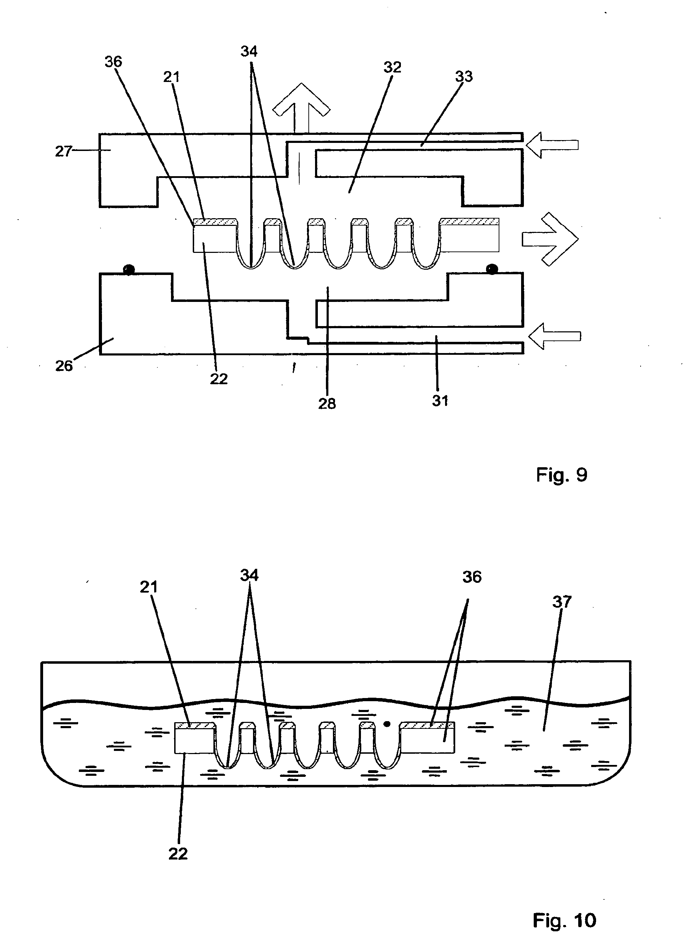

Fig. 9 zeigt einen sechsten Schritt der Ausführungsform des Verfahrens. Bei diesem sechsten Schritt ist die Verformung der ersten Folie 21 vollständig abgeschlossen. Das in dem ersten Hohlraum 28 vorhandene technische Vakuum und der im zweiten Hohlraum 32 vorhandene Druckstoß wurden abgebaut. Weiterhin ist das Halbzeug 24 abgekühlt. Es erfolgt nunmehr über den ersten Kanal 31 und über den zweiten Kanal 33 eine Belüftung des ersten Hohlraumes 28 und des zweiten Hohlraumes 32. Die erste Formhälfte 26 und die zweite Formhälfte 27 werden nunmehr zueinander geöffnet, sodass die Einheit bestehend aus der ersten Folie 21 mit den verformten Bereichen 34 und der zweiten Folie 22, welche nunmehr einen mikrostrukturierten Formkörper 36 bilden, entnommen werden kann.

Fig. 10 zeigt einen siebten Schritt der Ausführungsform des Verfahrens. Bei diesem siebten Schritt wird der Formkörper 36 in ein Ätzbad 37 getaucht. Bei dem Ätzbad 37 handelt es sich um eine 5-molare Natriumlauge mit einem 10%igen Anteil an Methanol, welche eine Temperatur von 45°C aufweist. Der Ätzvorgang führt dazu, dass die Ionendurchschüsse in der ersten Folie 21 ausgeätzt werden. Das Ausätzen führt insbesondere in den verformten Bereichen 34, in welchen die erste Folie 21 eine geringe Dicke aufweist, zu durchgängigen Poren 11 (gezeigt in Fig. 2).

Der Durchmesser der entstehenden Poren 11 kann über die Ätzzeit und über die Zusammensetzung des Ätzbades 37 eingestellt werden. Bei einer Dicke der ersten Folie 21 von 50 µm entstehen Poren 11 mit einem Durchmesser von 2 µm bei einer Ätzzeit von 30 min.

Nach dem Ätzen ist der mikrostrukturierte Formkörper 36 aus dem Ätzbad 37 herauszunehmen und zu spülen. Der mikrostrukturierte Formkörper 36 weist nunmehr in den verformten Bereichen 34 die Poren 11 auf, während die übrigen Bereiche undurchlässig sind.

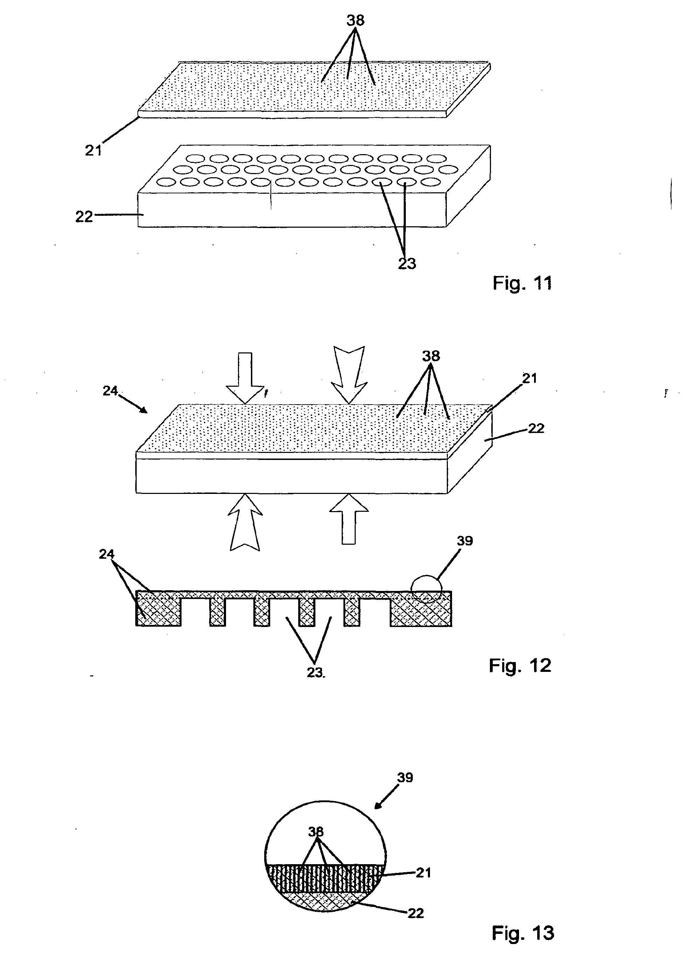

Fig. 11 zeigt einen ersten Schritt einer ersten bevorzugten Ausführungsform des erfindungsgemäßen Verfahrens, welche gegenüber der oben beschriebenen Ausführungsform abgewandelt ist. Im ersten Schritt werden wiederum die erste Folie 21 und die zweite Folie 22 bereitgestellt. Im Unterschied zu der in Fig. 4 gezeigten ersten Folie 21 weist die in Fig. 11 gezeigte erste Folie 21 nicht lediglich Ionendurchschüsse, sondern vollständige Poren 38 auf. Die Poren 38 können beispielsweise wie durch einen in Fig. 10 gezeigten Ätzvorgang geschaffen werden, nachdem die erste Folie 21 mit Schwerionen durchschossen wurde. Die Poren 38 können aber auch durch einen anderen Bearbeitungsvorgang in die erste Folie 21 eingebracht worden sein.

Fig. 12 zeigt einen zweiten Schritt der ersten bevorzugten Ausführungsform des erfindungsgemäßen Verfahrens, bei welchem die erste Folie 21 und die zweite Folie 22 durch ein Diffusionsschweißen miteinander verbunden werden. Dieser Diffusionsschweißvorgang gleicht dem in Fig. 5 gezeigten Diffusionsschweißvorgang. Ein Kreis kennzeichnet ein Detail 39 des Halbzeuges 24, welches in Fig. 13 gezeigt ist.

Fig. 13 zeigt das in Fig. 12 gezeigte Detail 39 des Halbzeuges 24. Die erste Folie 21 ist mit der zweiten Folie 22 fest verbunden, wodurch die Poren 38 in diesem Bereich verschlossen sind.

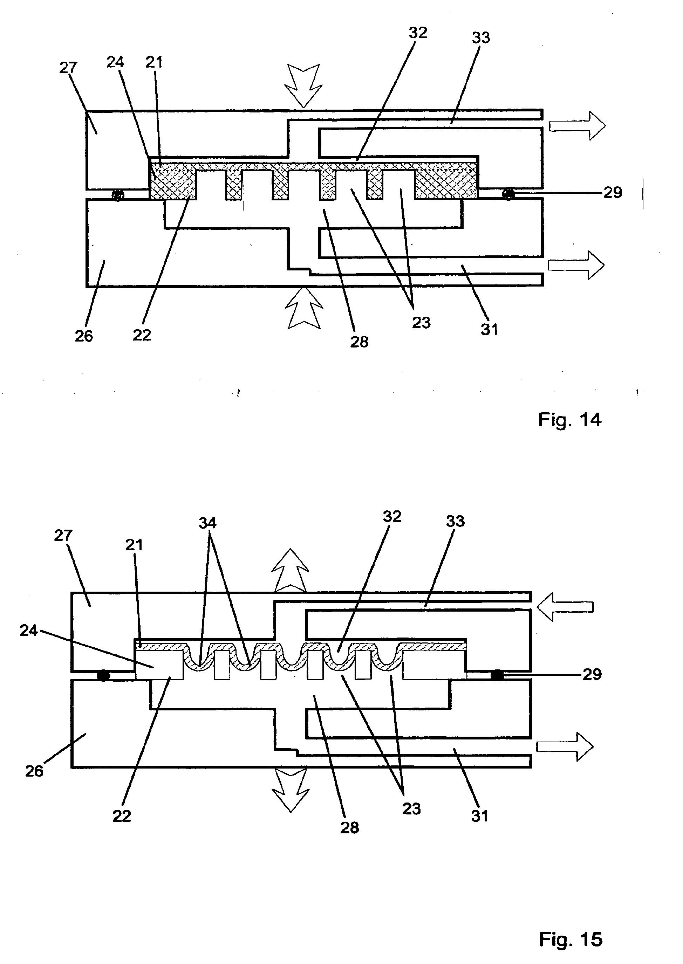

Fig. 14 zeigt einen dritten Schritt der ersten bevorzugten Ausführungsform des erfindungsgemäßen Verfahrens. Bei diesem dritten Schritt wird das Halbzeug 24 zwischen der ersten Formhälfte und der zweiten Formhälfte 27 angeordnet. Dieser Vorgang gleicht dem im Fig. 6 gezeigten Schritt.

Fig. 15 zeigt einen vierten Schritt der ersten bevorzugten Ausführungsform des erfindungsgemäßen Verfahrens, welcher dem in Fig. 7 gezeigten Schritt gleicht.

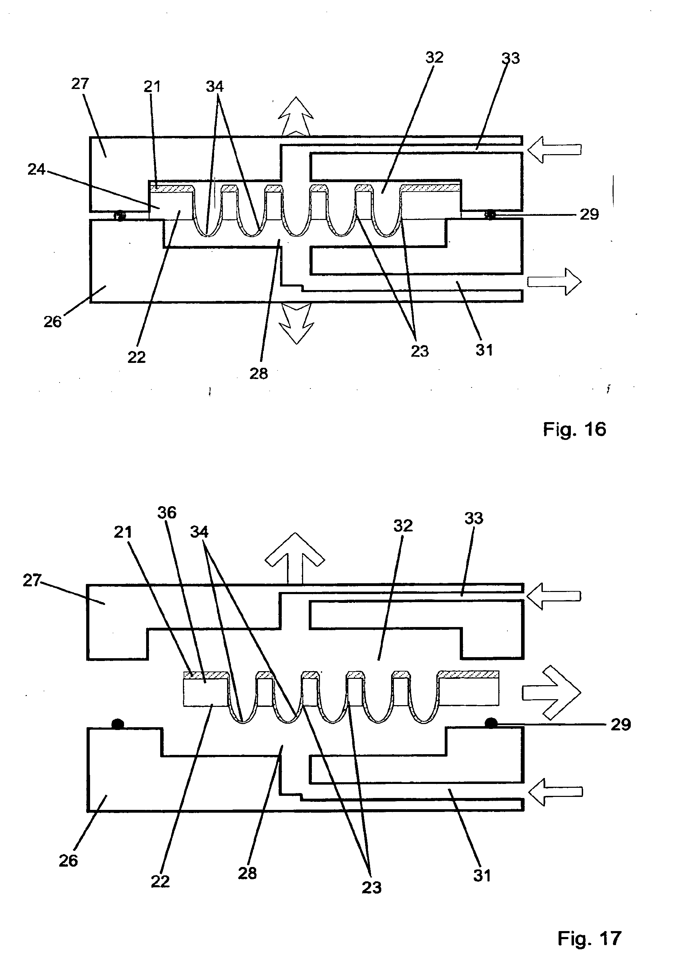

Fig. 16 zeigt einen fünften Schritt der ersten bevorzugten Ausführungsform des erfindungsgemäßen Verfahrens, welcher dem in Fig. 8 gezeigten Schritt gleicht. Die Poren 38 (gezeigt in Fig. 13) in der ersten Folie 21 führen dazu, dass Luft aus dem zweiten Hohlraum 32 in den ersten Hohlraum 28 gelangt, wodurch der Druckunterschied zwischen dem ersten Hohlraum 28 und dem zweiten Hohlraum 32 verringert wird. Diese Verringerung des Druckunterschiedes erfolgt aufgrund des kleinen Durchmessers der Poren 38 jedoch sehr langsam, sodass sie keinen wesentlichen Einfluss auf die Verformung der zu verformenden Bereiche 34 der ersten Folie 21 hat.

Fig. 17 zeigt einen sechsten Schritt der ersten bevorzugten Ausführungsform des erfindungsgemäßen Verfahrens, welcher dem in Fig. 9 gezeigten Schritt gleicht. Im Unterschied zur oben beschriebenen Ausführungsform ist die erste bevorzugte Ausführungsform des erfindungsgemäßen Verfahrens durch diesen sechsten Schritt abgeschlossen. Der mikrostrukturierte Formkörper 36 weist in den verformten Bereichen 34 bereits die Poren 38 (gezeigt in Fig. 13) auf und ist somit vollständig ausgebildet.

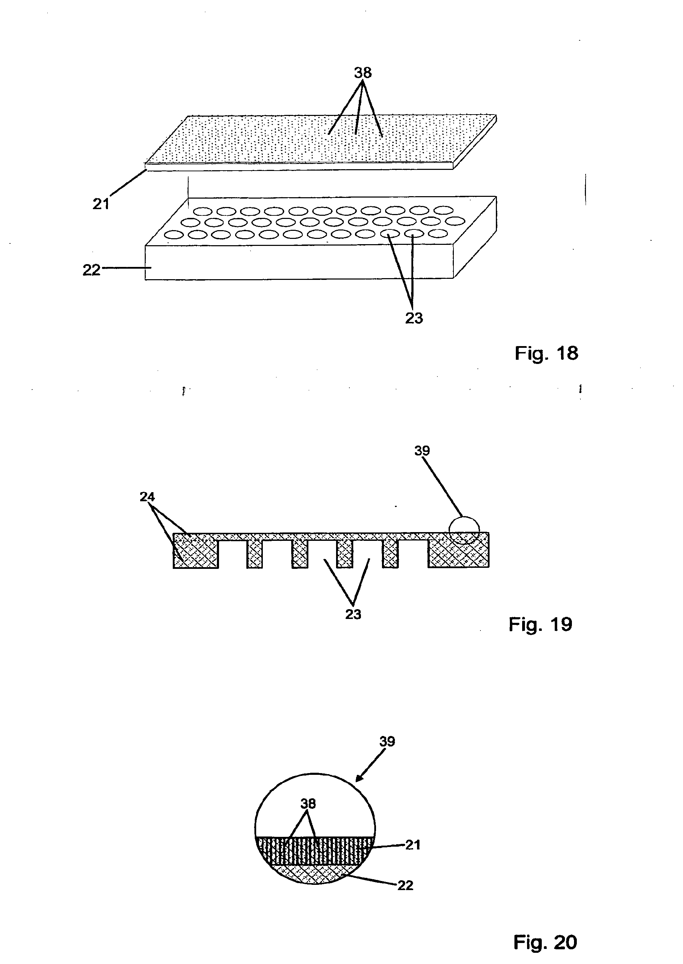

Fig. 18 zeigt einen ersten Schritt einer zweiten bevorzugten Ausführungsform des erfindungsgemäßen Verfahrens, welcher dem in Fig. 11 gezeigten Schritt gleicht. Die erste Folie 21 weist wiederum bereits die Poren 38 auf.

Fig. 19 zeigt einen zweiten Schritt der zweiten bevorzugten Ausführungsform des erfindungsgemäßen Verfahrens, welcher dem in Fig. 12 gezeigten Schritt gleicht.

Fig. 20 zeigt das Detail 39 des in Fig. 19 gezeigten Halbzeugs 24. Die Poren 38 in der ersten Folie 21 sind durch die zweite Folie 22 verschlossen.

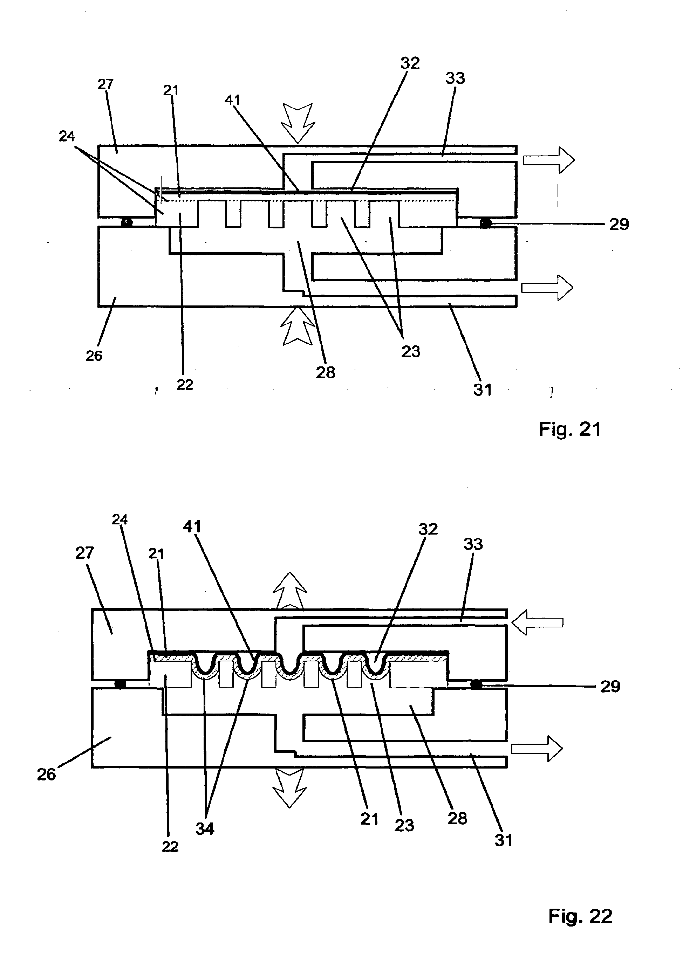

Fig. 21 zeigt einen dritten Schritt der zweiten bevorzugten Ausführungsform des erfindungsgemäßen Verfahrens, bei welchem das Halbzeug 24 zwischen die erste Formhälfte und die zweite Formhälfte 27 eingelegt wird. Dieser Schritt gleicht zunächst dem in Fig. 14 gezeigten Schritt. Im Gegensatz zu der ersten bevorzugten Ausführungsform des erfindungsgemäßen Verfahrens wird zwischen der ersten Folie 21 und der zweiten Formhälfte 27 zusätzlich eine Opferfolie 41 angeordnet, welche die erste Folie 21 vollständig bedeckt. Die Opferfolie 41 besteht aus demselben Material wie die zweite Folie 22 und weist eine Dicke zwischen etwa 50 µm und 100 µm auf. Die Opferfolie 41 dient insbesondere dazu, die Poren 38 (gezeigt in Fig. 18) während der Verformung zwischen der ersten Formhälfte 26 und der zweiten Formhälfte 27 abzudichten.

Fig. 22 zeigt einen vierten Schritt der zweiten bevorzugten Ausführungsform des erfindungsgemäßen Verfahrens, welcher zunächst dem in Fig. 15 gezeigten Schritt gleicht. Im Unterschied dazu wird weiterhin die Opferfolie 41 in gleicher Weise wie die erste Folie 21 verformt. Die Opferfolie 41 gewährleistet, dass es nicht zu einem Druckausgleich zwischen dem zweiten Hohlraum 32 und dem ersten Hohlraum 28 kommt. Auch verhindert die Opferfolie 41, dass es zu Durchbrüchen in den verformten Bereichen 34 der ersten Folie 21 kommt, sodass der Druck des Druckstoßes höher als bei der ersten bevorzugten Ausführungsform gewählt werden kann.

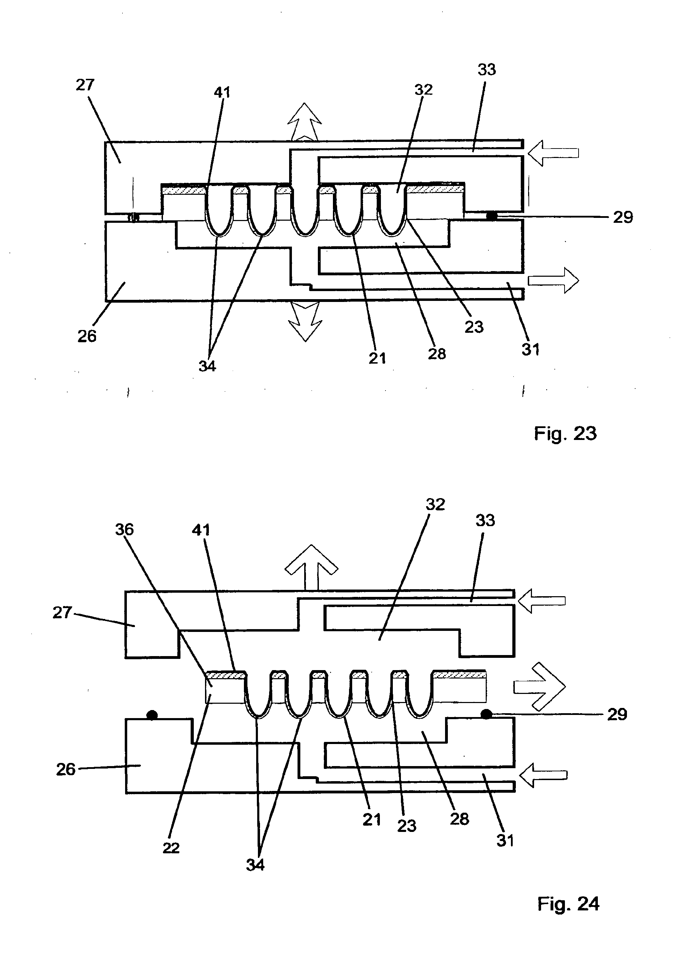

Fig. 23 zeigt einen fünften Schritt der zweiten bevorzugten Ausführungsform des erfindungsgemäßen Verfahrens, welcher zunächst dem in Fig. 16 gezeigten Schritt gleicht. Im Unterschied dazu folgt auch die Opferfolie 41 der fortgesetzten Verformung der ersten Folie 21.

Fig. 24 zeigt einen sechsten Schritt der zweiten bevorzugten Ausführungsform des erfindungsgemäßen Verfahrens, welcher zunächst dem in Fig. 17 gezeigten Schritt gleicht. Im Unterschied dazu befindet sich die Opferfolie 41 weiterhin am mikrostrukturierten Formkörper 36.

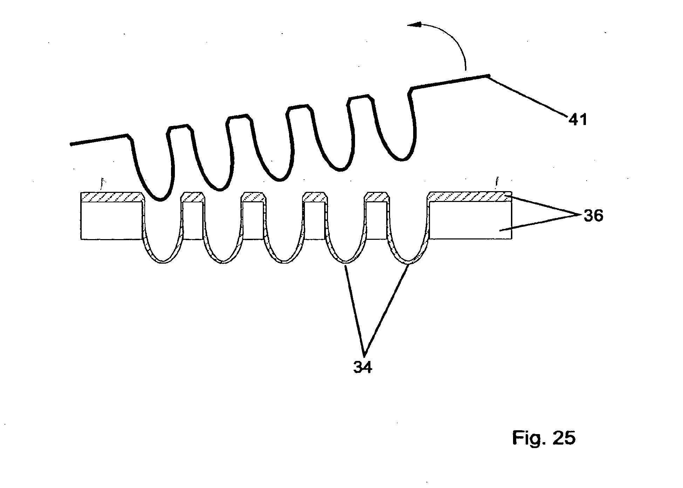

Fig. 25 zeigt einen siebten Schritt der zweiten bevorzugten Ausführungsform des erfindungsgemäßen Verfahrens. Bei diesem siebten Schritt wird die Opferfolie 41 vom mikrostrukturierten Formkörper 36 getrennt. Damit liegt der mikrostrukturierte Formkörper 36 mit Poren 38 (gezeigt in Fig. 18) in den verformten Bereichen 34 der ersten Folie 21 vor, wodurch die Herstellung abgeschlossen ist.

### Bezugszeichenliste

- 01: erste Folienschicht
- 02: zweite Folienschicht
- 03: durchgängige Ausnehmung
- 04: verformter Bereich
- 05: -
- 06: Kavität
- 07: verformter Bereich
- 08: unverformter Bereich
- 09: unverformter Teilbereich
- 10: -
- 11: Poren
- 12: Krater
- 21: erste Folie
- 22: zweite Folie
- 23: Ausnehmungen
- 24: Halbzeug
- 25: -
- 26: erste Formhälfte
- 27: zweite Formhälfte
- 28: erster Hohlraum
- 29: Dichtelemente
- 31: erster Kanal
- 32: zweiter Hohlraum
- 33: zweiter Kanal
- 34: verformte Bereiche
- 36: mikrostrukturierter Formkörper
- 37: Ätzbad
- 38: Poren
- 39: Detail des Halbzeuges
- 40: -
- 41: Opferfolie

## Patentansprüche

1. Verfahren zur Herstellung eines mikrostrukturierten Formkörpers (01, 02; 36) mit perforierten Teilen (07), die folgenden Schritte umfassend:
- Bereitstellen einer verformbaren ersten Folie (01; 21) mit einer Dicke von weniger als 1 mm;
- Bereitstellen einer zweiten Folie (02; 22), welche Ausnehmungen (03; 23) aufweist, deren Durchmesser kleiner als 2 mm sind;
- Erzeugen von Poren (11; 38) in der ersten Folie (01; 21), wobei der Durchmesser der Poren (11; 38) zwischen 10 nm und 10 µm beträgt;
- Anordnen der ersten Folie (01; 21) über der zweiten Folie (02; 22);
- Verformen von Bereichen (04; 34) der ersten Folie (01; 21) in die Ausnehmungen (03; 23) der zweiten Folie (02; 22), wodurch die verformten Bereiche (04; 34) Kavitäten (06) ausbilden; und
- Verbinden der ersten Folie (01; 21) mit der zweiten Folie (02; 22) zu einem einzigen mikrostrukturierten Formkörper (01, 02; 36), wobei das Verbinden der ersten Folie (01; 21) mit der zweiten Folie (02; 22) durch ein Diffusionsschweißen, durch ein Laser-Schweißen, durch ein Ultraschallschweißen oder durch ein Laminieren mit einer dazwischen befindlichen Klebeschicht erfolgt;
**dadurch gekennzeichnet, dass** das Erzeugen der Poren (11; 38) vor dem Verformen der ersten Folie (01; 21) in den zu verformenden Bereichen (04; 34) und in nicht zu verformenden Bereichen (08) der ersten Folie (01; 21) erfolgt, wobei die Poren (11; 38) in den nicht zu verformenden Bereichen (08) der ersten Folie (01; 21) durch das Verbinden der ersten Folie (01; 21) mit der zweiten Folie (02; 22) verschlossen werden.

2. Verfahren nach Anspruch 1, **dadurch gekennzeichnet, dass** das Anordnen der ersten Folie (01; 21) über der zweiten Folie (02; 22), das Verformen der ersten Folie (01; 21) und das Verbinden der ersten Folie (01; 21) mit der zweiten Folie (02; 22) die folgenden Teilschritte umfasst:
- Verwenden eines Thermoplasts als Material für die erste Folie (01; 21);
- Verwenden eines Materials für die zweite Folie (02; 22), welches bei einer Glasübergangstemperatur des Materials der ersten Folie (01; 21) unverformbar ist;
- Anordnen der ersten Folie (01; 21) über der zweiten Folie (02; 22) zwischen einer ersten Formhälfte (26) und einer zweiten Formhälfte (27), wobei die erste Folie (01; 21) zwischen der zweiten Formhälfte (27) und der zweiten Folie (02; 22) angeordnet ist;
- Erwärmen der ersten Folie (01; 21) bis mindestens zu der Glasübergangstemperatur des Thermoplasts der ersten Folie (01; 21);
- Erzeugen eines Unterdruckes zwischen der ersten Formhälfte (26) und der zweiten Folie (02; 22), der durch die Ausnehmungen (03; 23) in der zweiten Folie (02; 22) auch die zu verformenden Bereiche (04; 34) der ersten Folie (01; 21) beaufschlagt, wodurch die zu verformenden Bereiche (04; 34) der ersten Folie (01; 21) in die Ausnehmungen (03; 23) in der zweiten Folie (02; 22) geformt werden;
- Zusammenpressen der ersten Formhälfte (26) und der zweiten Formhälfte (27) mit den beiden dazwischen befindlichen Folien (01, 02; 21, 22), wodurch die beiden Folien (01, 02; 21, 22) durch einen Diffusionsschweißvorgang miteinander verbunden werden;
- Abkühlen der beiden Folien (01, 02; 21, 22), wodurch ein einziger mikrostrukturierter Formkörper (01, 02; 36) geschaffen wird; und
- Herausnehmen des mikrostrukturierter Formkörpers (01, 02; 36) aus den beiden Formhälften (26, 27).

3. Verfahren nach Anspruch 2, **dadurch gekennzeichnet, dass** beim Anordnen der ersten Folie (01; 21) über der zweiten Folie (02; 22) zwischen der ersten Formhälfte (26) und der zweiten Formhälfte (27) weiterhin eine zu verformende Opferfolie (41) zwischen der ersten Folie (01; 21) und der zweiten Formhälfte (27) angeordnet wird, wobei die Opferfolie (41) nach dem Herausnehmen des mikrostrukturierter Formkörper (01, 02; 36) aus den beiden Formhälften (26, 27) von dem mikrostrukturierter Formkörper (01, 02; 36) getrennt wird.

4. Mikrostrukturierter Formkörper (01, 02; 36), herstellbar gemäß einem Verfahren nach einem der Ansprüche 1 bis 3, umfassend einen folienartigen Grundkörper (01, 02; 36), der eine erste Folienschicht (01; 21) und eine darunter befindliche zweite Folienschicht (02; 22) umfasst, wobei die zweite Folienschicht (02; 22) Ausnehmungen (03; 23) mit einem Durchmesser von weniger als 2 mm aufweist, die durch verformte Bereiche (04; 34) der ersten Folienschicht (01; 21) ausgeformt sind, durch welche Kavitäten (06) ausgebildet sind, wobei zumindest einige der verformten Bereiche (04; 34) und einige der unverformten Bereiche (08) der ersten Folienschicht (01; 21) Poren (11; 38) aufweisen, und wobei Bereiche (08) des folienartigen Grundkörpers (01, 02; 36) außerhalb der Ausnehmungen (03; 23) undurchlässig sind, indem die Poren (11; 38) in den nicht verformten Bereichen (08) der ersten Folienschicht (01; 21) durch die zweite Folienschicht (02; 22) verschlossen sind.

5. Mikrostrukturierter Formkörper (01, 02; 36) nach Anspruch 4, **dadurch gekennzeichnet, dass** die Tiefe der Kavitäten (06) jeweils mehr als doppelt so groß wie der Durchmesser der durch die jeweilige Kavität (06) ausgeformten Ausnehmung (03; 23) ist.

6. Mikrostrukturierter Formkörper (01, 02; 36) nach Anspruch 4 oder 5, **dadurch gekennzeichnet, dass** die verformten Bereiche (04; 34) der ersten Folienschicht (01; 21) durch die Ausnehmungen (03; 23) in der zweiten Folienschicht (02; 22) hindurchragen und aus diesen herausragen.

## Claims

1. A method for the manufacture of a micro-structured moulded body (01, 02; 36) with perforated parts (07), comprising the following steps:
- provision of a deformable first film (01; 21) with a thickness of less than 1 mm;
- provision of a second film (02; 22), which has openings (03; 23), whose diameters are smaller than 2 mm;
- generation of pores (11; 38) in the first film (01; 21), wherein the diameter of the pores (11; 38) is between 10 nm and 10 µm;
- arrangement of the first film (01; 21) above the second film (02; 22);
- deformation of regions (04; 34) of the first film (01; 21) into the openings (03; 23) of the second film (02; 22), whereby the deformed regions (04; 34) form cavities (06); and
- joining of the first film (01; 21) with the second film (02; 22) to form a single micro-structured moulded body (01, 02; 36), wherein the joining of the first film (01; 21) with the second film (02; 22) takes place by means of diffusion welding, by means of laser welding, by means of ultrasound welding, or by means of a lamination process with an adhesive layer located between the two films;
**characterised in that** the generation of the pores (11; 38) takes place before the deformation of the first film (01; 21) into the regions (04; 34) that are to be deformed, and in regions (08) of the first film (01; 21) that are not to be deformed, wherein the pores (11; 38) in the regions (08) of the first film (01; 21) that are not to be deformed are closed by the joining of the first film (01; 21) with the second film (02; 22).

2. The method in accordance with claim 1, **characterised in that** the arrangement of the first film (01; 21) above the second film (02; 22), the deformation of the first film (01; 21), and the joining of the first film (01; 21) with the second film (02; 22) comprise the following sub-steps:
- use of a thermoplastic as the material for the first film (01; 21);
- use of a material for the second film (02; 22), which is non-deformable at a glass transition temperature of the material of the first film (01; 21);
- arrangement of the first film (01; 21) above the second film (02; 22) between a first mould half (26) and a second mould half (27), wherein the first film (01; 21) is arranged between the second mould half (27) and the second film (02; 22);
- heating of the first film (01; 21) up to at least the glass transition temperature of the thermoplastic of the first film (01; 21);
- generation of a reduced pressure between the first mould half (26) and the second film (02; 22), which through the openings (03; 23) in the second film (02; 22) is also applied to the regions (04; 34) of the first film (01; 21) that are to be deformed, whereby the regions (04; 34) of the first film (01; 21) that are to be deformed are shaped into the openings (03; 23) in the second film (02; 22);
- compression of the first mould half (26) and the second mould half (27), with the two films (01, 02; 21, 22) located between them, whereby the two films (01, 02; 21, 22) are joined with one another by means of a diffusion welding process;
- cooling of the two film (01, 02; 21, 22), whereby a single micro-structured moulded body (01, 02; 36) is created; and
- removal of the micro-structured moulded body (01, 02; 36) from the two mould halves (26; 27).

3. The method in accordance with claim 2, **characterised in that** in the arrangement of the first film (01; 21) above the second film (02; 22) between the first mould half (26) and the second mould half (27) a sacrificial film (41) that is to be deformed is also arranged between the first film (01; 21) and the second film (27), wherein after the removal of the micro-structured moulded body (01, 02; 36) from the two mould halves (26, 27) the sacrificial film (41) is separated from the micro-structured moulded body (01, 02; 36).

4. A micro-structured moulded body (01, 02, 36), which can be manufactured in accordance with a method in accordance with one of the claims 1 to 3, comprising a film-type base body (01, 02, 36) which comprises a first film layer (01, 21) and a thereunder located second film layer (02, 22), wherein the second film layer (02, 22) has openings (03, 23) with a diameter of less than 2 mm, which are shaped by means of deformed regions (04, 34) of the first film layer (01, 21), by means of which cavities (06) are formed, wherein at least some of the deformed regions (04, 34) and some of the non-deformed regions (08) of the first film layer (01, 21) have pores (11, 38), and wherein regions (08) of the film-type base body (01, 02, 36) outboard of the openings (03, 23) are non-permeable, in that the pores (11, 38) in the non-deformed regions (08) of the first film layer (01, 21) are closed by means of the second film layer (02, 22).

5. The micro-structured moulded body (01, 02, 36) in accordance with claim 4, **characterised in that** the depth of the cavities (06) is in each case more than twice as large as the diameter of the opening (03, 23) shaped by means of the respective cavity (06).

6. The micro-structured moulded body (01, 02, 36) in accordance with claim 4 or 5, **characterised in that** the deformed regions (04, 34) of the first film layer (01, 21) protrude through the openings (03, 23) in the second film layer (02, 22), and protrude beyond the latter.

## Revendications

1. Procédé de fabrication d'un corps de moule microstructuré (01, 02 ; 36) comprenant des parties perforées (07), comprenant les étapes suivantes :
- préparation d'un premier film (01 ; 21) déformable ayant une épaisseur inférieure à 1 mm ;
- préparation d'un second film (02 ; 22) qui présente des évidements (03 ; 23) dont le diamètre est inférieur à 2 mm ;
- création de pores (11 ; 38) dans le premier film (01 ; 21), sachant que le diamètre des pores (11 ; 38) fait entre 10 nm et 10 µm ;
- agencement du premier film (01 ; 21) sur le second film (02 ; 22),
- déformation de parties (04 ; 34) du premier film (01 ; 21) dans les évidements (03 ; 23) du second film (02 ; 22), ce par quoi les parties (04 ; 34) déformées forment des cavités (06) ; et
- liaison du premier film (01 ; 21) au second film (02 ; 22) en un corps de moule microstructuré unique (01, 02 ; 36), sachant que la liaison du premier film (01 ; 21) au second film (02 ; 22) est effectuée par un soudage par diffusion, par un soudage au laser, par un soudage aux ultrasons ou par un laminage avec une couche de colle intercalée ;
**caractérisé en ce que** la création des pores (11 ; 38) est effectuée avant la déformation du premier film (01 ; 21) dans les parties (04 ; 34) à déformer et dans des parties (08) du premier film (01 ; 21) qui ne sont pas à déformer, sachant que les pores (11 ; 38) dans les parties (08) du premier film (01 ; 21) qui ne sont pas à déformer sont fermés par la liaison du premier film (01 ; 21) au second film (02 ; 22).

2. Procédé selon la revendication 1, **caractérisé en ce que** l'agencement du premier film (01 ; 21) sur le second film (02 ; 22), la déformation du premier film (01 ; 21) et la liaison du premier film (01 ; 21) au second film (02 ; 22) comprennent les étapes partielles suivantes :
- emploi d'un thermoplastique en tant que matériau pour le premier film (01 ; 21) ;
- emploi d'un matériau pour le second film (02 ; 22), lequel est indéformable à une température de transition vitreuse du matériau du premier film (01 ; 21) ;
- agencement du premier film (01 ; 21) sur le second film (02 ; 22) entre une première moitié de moule (26) et une seconde moitié de moule (27), sachant que le premier film (01 ; 21) est agencé entre la seconde moitié de moule (27) et le second film (02 ; 22) ;
- chauffage du premier film (01 ; 21) jusqu'à au moins la température de transition vitreuse du thermoplastique du premier film (01 ; 21) ;
- production d'une dépression entre la première moitié de moule (26) et le second film (02 ; 22) qui touche également les parties (04 ; 34) à déformer du premier film (01 ; 21) par les évidements (03 ; 23) dans le second film (02 ; 22), ce par quoi les parties (04 ; 34) à déformer du premier film (01 ; 21) sont formées dans les évidements (03 ; 23) dans le second film (02 ; 22) ;
- compression de la première moitié de moule (26) et de la seconde moitié de moule (27) avec les deux films (01, 02 ; 21, 22) se trouvant entre, ce par quoi les deux films (01, 02 ; 21, 22) sont reliés entre eux par un procédé de soudure par diffusion ;
- refroidissement des deux films (01, 02 ; 21, 22), ce par quoi un corps de moule microstructuré unique (01, 02 ; 36) est formé ; et
- retrait du corps de moule microstructuré unique (01, 02 ; 36) des deux moitiés de moule (26, 27).

3. Procédé selon la revendication 2, **caractérisé en ce que** lors de l'agencement du premier film (01 ; 21) sur le second film (02 ; 22) entre la première moitié de moule (26) et la seconde moitié de moule (27), un film sacrifié (41) à déformer est placé en outre entre le premier film (01 ; 21) et la seconde moitié de moule (27), sachant que le film sacrifié (41) est séparé du corps de moule microstructuré unique (01, 02 ; 36) après le retrait du corps de moule microstructuré unique (01, 02 ; 36) des deux moitiés de moule (26, 27).

4. Corps de moule microstructuré unique (01, 02 ; 36), pouvant être fabriqué selon un procédé selon l'une des revendications 1 à 3, comprenant un corps de base de type film (01, 02 ; 36) qui comprend une première couche de film (01 ; 21) et une seconde couche de film (02 ; 22) se trouvant dessous, sachant que la seconde couche de film (02 ; 22) présente des évidements (03 ; 23) d'un diamètre inférieur à 2 mm, qui sont formés par des parties (04 ; 34) déformées de la première couche de film (01 ; 21), par lesquels des cavités (06) sont formées, sachant qu'au moins quelques-unes des parties (04 ; 34) déformées et quelques-unes des parties non déformées (08) de la première couche de film (01 ; 21) présentent des pores (11 ; 38), et sachant que des parties (08) du corps de base (01, 02 ; 36) de type couche sont imperméables en-dehors des évidements (03 ; 23), en ce que les pores (11 ; 38) dans les parties non déformées (08) de la première couche de film (01 ; 21) sont fermés par la seconde couche de film (02 ; 22).

5. Corps de moule microstructuré unique (01, 02 ; 36) selon la revendication 4, **caractérisé en ce que** la profondeur des cavités (06) fait respectivement plus du double du diamètre de l'évidement (03 ; 23) formé par la cavité (06) respective.

6. Corps de moule microstructuré unique (01, 02 ; 36) selon la revendication 4 ou 5, **caractérisé en ce que** les parties déformées (04 ; 34) de la première couche de film (01 ; 21) font saillie à travers les évidements (03 ; 23) dans la seconde couche de film (02 ; 22) et hors de celle-ci.
